Europäisches Patentamt

European Patent Office (11) Publication number: **0 387 095**

Office européen des brevets **A1**

## (12) EUROPEAN PATENT APPLICATION

(21) Application number: **90302556.7**

(22) Date of filing: **09.03.90**

(51) Int. Cl.⁵: **C12P 21/08, A61K 39/395**

(30) Priority: **09.03.89 GB 8905400**

(43) Date of publication of application:
**12.09.90 Bulletin 90/37**

(84) Designated Contracting States:
**AT BE CH DE DK ES FR GB GR IT LI LU NL SE**

(71) Applicant: **Jonker, Margreet**
**Hooiweide 3**
**NL-2353 MR Leiderdorp(NL)**

Applicant: **Van Der Meide, Petrus Hendrikus**
**Kastanjelaan 2**
**NL-2631 HT Nootdorp(NL)**

(72) Inventor: **Jonker, Margreet**
**Hooiweide 3**
**NL-2353 MR Leiderdorp(NL)**
Inventor: **Van Der Meide, Petrus Hendrikus**
**Kastanjelaan 2**
**NL-2631 HT Nootdorp(NL)**

(74) Representative: **Hallybone, Huw George et al**
**CARPMAELS AND RANSFORD 43**
**Bloomsbury Square**
**London WC1A 2RA(GB)**

(54) **Pharmaceutical product for the treatment of immunoregulatory disorders.**

(57) A pharmaceutical product comprises an antibody to tumour necrosis factor and an antibody to interferon-γ as a combined preparation for simultaneous separate or sequential use. The product is particularly suitable for treating immunoregulatory disorder such as organ transplant rejection or graft rejection. While antibody to tumour necrosis factor or antibody to interferon-γ alone have little or no effect on rejection, the combination of antibodies produces a surprising prolongation of graft or transplant survival.

EP 0 387 095 A1

## Pharmaceutical Product for the Treatment of Immunoregulatory Disorders

Technical Field

This invention relates to a pharmaceutical product, which is particularly useful for the treatment of immunoregulatory disorders, and to the production and use of such a product. In particular, it relates to products comprising an antibody to tumour necrosis factor (TNF).

Background Art

Cytokines, of which tumour necrosis factor (TNF) and interferon-$\gamma$ (IFN-$\gamma$) are examples, are known to play an important regulatory role in the network of cellular interactions which constitute the immune response. For example, they are involved in the recruitment of immunocompetent cells into the site of inflammation and the amplification of the immune response, in which major histocompatibility complex (MHC) antigens play a key role.

INF-$\gamma$ is known to exert a wide variety of immunomodulatory effects. It mediates the chemotactic migration and activation of T cells (Issekutz, T.B., et al., J. Immunol 1988, 140: 2989) and macrophages (Nathan, C.F., et al., N. Engl. J. Med 1986, 315:6) and enhances the cytotoxic activity of natural killer cells, monocytes, cytotoxic T cells and polymorphonuclear leucocytes (Trinchieri, G. et al., J. Exp. Med 1984, 160: 1147). Furthermore, IFN-$\gamma$ enhances the expression of MHC class I and II antigens on almost every cell type both in vitro (Fellous, M., et al., PNAS 1982, 79: 3082; Wong, G.H.W., et al., Eur. J. Immunol 1984, 14: 52) and in vivo (Momburg, F., et al., Eur. J. Immunol 1986, 16: 551); Steiniger, B., et al., Transpl. Proc, 1987, XIX(5): 4322) and it induces more de novo synthesis of receptors for other cytokines such as TNF (Ruggiero, V., et al., J. Immunol 1986, 136, 2445).

Like IFN-$\gamma$, TNF increases the expression of antigenic structures such as ICAM-1, relevant in the adhesion of circulating granulocytes, moncytes and lymphocytes to endothelial cells (Pober, J.S., et al J. Immunol 1986, 137: 1893). Moreover, TNF exerts direct cytotoxic effects towards endothelial cells (Sato, N., et al J.N.C.I. 1986, 76: 1113) and is involved in the regulation of both MHC class I and II antigen expression (Collins T., et al, PNAS, 1985, 83: 446).

The MHC antigens are known to play a critical role in the development and augmentation of the immune response by which the host tries to reject non-self tissue (see for example Milton, A.D. et al., J. Exp. Med 1985, 161: 98). Thus, it might be expected that an antibody specific for INF-$\gamma$ or TNF would be of use in the treatment of immunoregulatory disorders in which rejection of self or non-self tissue occurs, for example, in autoimmune diseases or organ transplant rejection.

Antibodies to INF-$\gamma$ and TNF are already known and the antibodies have been disclosed in combination (Sandru, G., et al., Cancer Res. (1988) 48, 5411). This reference describes in vitro experiments designed to investigate a mononuclear cell derived factor which appears to stimulate tumour cell growth. The reference describes a synergistic stimulatory effect on tumour cell growth when anti-Ifn-$\gamma$ and anti-TNF were supplied together. This paper therefore suggests that the combination antibodies of would not be useful in therapy. Furthermore, there is no suggestion in the paper that the antibodies might be useful for the treatment of immunoregulatory disorders.

Disclosure of the Invention

Despite their expectations that antibodies specific for IFN-$\gamma$ or TNF would be of use in the treatment of immunoregulatory disorders, in tests using a rhesus monkey skin transplantation model, the present inventors have found that administration of anti-IFN-$\gamma$ antibody to the animal prior to skin transplantation has no detectable effect on the survival of the skin graft. Similarly, administration of anti-TNF antibody alone, does not prolong skin graft survival. Surprisingly, however, the inventors have found that co-administration of an anti-IFN-$\gamma$ antibody and an anti-TNF antibody results in a prolongation of graft survival times, and have used this to develop a means for the treatment of immunoregulatory disorders.

Thus, according to one aspect of the present invention there is provided a pharmaceutical product containing an antibody to tumour necrosis factor and an antibody to interferon-$\gamma$ as a combined preparation for simultaneous separate or sequential use in therapy, and, in particular, in the therapy of an immunoregulatory disorder. The pharmaceutical product may, for example comprise the antibodies in admixture optionally together with a pharmaceutical acceptable excipient, diluent or carrier. Preferably, the pharmaceutical product is suitable for administration to the human body.

The term tumour necrosis factor (TNF) as used herein is intended to mean, in particular, human TNF, and, especially, human $\alpha$-TNF. Similarly, the term, interferon-$\gamma$ (IFN-$\gamma$) as used herein is intended to mean, in particular, human IFN-$\gamma$.

The term immunoregulatory disorder as used herein is to be understood to encompass any rejection episode following an organ or tissue transplant (for example rejection of a skin graft) or an autoimmune disease, e.g. organ specific disease such as thyroiditis or non-organ specific disease such as rheumatoid arthritis.

The antibody against tumour necrosis factor (hereinafter referred to as anti-TNF antibody) and the antibody against interferon gamma (hereinafter referred to as anti-IFN-$\gamma$ antibody) may each be a whole antibody or an antibody fragment. Antibody fragments include for example fragments derived by proteolytic cleavage of a whole antibody, such as F(ab')$_2$, Fab' or Fab fragments, or fragments obtained by recombinant DNA techniques, for example Fv fragments (as described in International Patent Application No. WO89/02465

Each anti-TNF or anti-IFN-$\gamma$ antibody or antibody fragment may in general belong to any immunoglobulin class. Thus, for example, it may be an immunoglobulin M antibody or, in particular, an immunoglobulin G antibody. The antibody or fragment may be of animal, for example mammalian origin and may be for example of murine, rat or human origin. It may be a natural antibody or a fragment thereof, or, if desired, a recombinant antibody or antibody fragment, i.e. an antibody or antibody fragment which has been produced using recombinant DNA techniques.

Particular recombinant antibodies or antibody fragments include, (1) those having an antigen binding site at least part of which is derived from a different antibody, for example those in which the hypervariable or complementarity determining regions of one antibody, which may be of murine origin, have been grafted into the variable framework regions of a second, different antibody, which may be of human origin, (as described in European Patent Specification No. 239400); (2) recombinant antibodies or fragments wherein non-Fv sequences have been substituted by non-Fv sequences from other, different antibodies, which may be of human origin, (as described in European Patent Specifications Nos. 171496, 173494 and 194276); or (3) recombinant antibodies or fragments possessing substantially the structure of a natural immunoglobulin but wherein the hinge region has a different number of cysteine residues from that found in the natural immunoglobulin, or wherein one or more cysteine residues in a surface pocket of the recombinant antibody or fragment is in the place of another amino acid residue present in the natural immunoglobulin (as described in International Patent Applications Nos. WO89/01782 and WO89/01974 respectively)

The antibody or antibody fragment may be polyspecific, but is preferably monospecific, for tumour necrosis factor or interferon gamma. The antibody or fragment may be of polyclonal, or, preferably, monoclonal origin.

The antibodies for use in a composition according to the invention may be prepared using well-known immunological techniques employing either TNF or IFN-$\gamma$ as antigen. Thus, for example, any suitable host may be injected with TNF or IFN-$\gamma$ and the serum collected to yield the desired polyclonal anti-TNF or anti-IFN-$\gamma$ antibody after appropriate purification and/or concentration, (for example by affinity chromatography using immobilised TNF or IFN-$\gamma$ as the affinity medium). Alternatively, splenocytes or lymphocytes may be recovered from the TNF or IFN-$\gamma$ injected host and immortalised using for example the method of Kohler et al., Eur. J. Immunol. 6, 511, (1976), the resulting cells being segregated to obtain a single genetic line producing monoclonal anti-TNF or anti-IFN-$\gamma$ antibodies in accordance with conventional practice.

Antibody fragments may be produced using conventional techniques, for example by enzymatic digestion of whole antibodies e.g. with pepsin [Parham, J. Immunol., 131, 2895, (1983)] or papain [Lamoyi and Nisonoff, J. Immunol. Meth., 56, 235, (1983)]. Where it is desired to produce recombinant anti-TNF and anti-IFN-$\gamma$ antibodies these may be produced using for example the methods described in the above previously mentioned patent specifications.

In some embodiments, the pharmaceutical product according to the invention may contain another active ingredient.

The pharmaceutical product according to the invention may take any suitable form for administration, and in particular will be in a form suitable for patenteral administration, e.g. by injection or infusion. Suitable formulations for parenteral administration include suspensions, solutions or emulsions of both or each of the antibodies in oily or aqueous vehicles, optionally containing formulatory agents such as suspending, stabilising and/or dispersing agents. Alternatively, the antibodies may be in powder form for reconstitution with a suitable vehicle, e.g. sterile pyrogen-free water, before use. The pharmaceutical product may also, suitably, be formulated for topical administration for example to the site of a skin graft.

The quantities of each antibody present in the product according to the invention will in general depend on the intended use of the product and may vary according to such factors as the immunoregulatory disorder to be treated, the route of administration, and the age, sex and condition of the patient.

Thus the exact quantities of each antibody may need to be determined empirically, in accordance with conventional practice before each treatment regime is started. In general, however, each antibody may be administered at a dose in the range 0.1 - 5mg/kg/day, for example around 1 to around 2 mg/kg/day. The relative quantity of each antibody administered may be varied, for example in a ratio by weight ranging from around 1:1 to around 1:2.

The product according to the invention may be administered prophylatically, or after an immunoregulatory disorder has occurred. Thus, for example, the product may be administered before an organ or tissue transplant, or after an immunoregulatory disorder has occurred, and continued thereafter for as long as necessary.

According to a further aspect of the invention there is provided the use of an anti-TNF antibody and an anti-IFN-γ antibody as described above in the manufacture of a pharmaceutical product for use in the therapy of an immunoregulatory disorder. For example, the pharmaceutical product may be produced by arranging the anti-TNF antibody and the anti-IFN-γ antibody as a combined preparation for simultaneous separate or sequential use. Alternatively the pharmaceutical product may be produced by mixing an anti-TNF antibody, an anti-IFN-γ antibody and, optionally, a pharmaceutically acceptable excipient, diluent or carrier.

In a still further aspect the invention provides a method of treatment of a human or animal subject (preferably, a human), the method comprising administering to the subject effective amounts of an anti-TNF anitbody and an anti-IFN-γ antibody.

## Mode for Carrying Out the Invention

The invention is illustrated below by way of example only.

The inventors have used a skin transplantation study in the rhesus monkey to investigate the immunosuppressive potencies of two murine monoclonal antibodies (MAbs), MD1 and 61E71, specific for human recombinant IFN-γ and human recombinant TNF-α respectively (Meide, P.H., et al., J. Immunol. Meth 1985, 79: 293; Collins T., et al., PNAS, 1985, 83: 446).

A study in primates was performed as MD1 and 61E71 are, respectively, cross reactive with IFN-γ and TNF-α of the rhesus monkey. Furthermore, as the rhesus monkey has a close phylogenetic relationship to the human being and the rhesus immune system shows great similarity to that of man, the results should be reliably extrapolated to the human situation.

## Materials and methods

### 1) Animals

Rhesus monkeys (Macaca mulatta) were born and raised in the TNO Primate Center, Rijswijk, employing a harem type breeding system. All monkeys were two to four years of age and typed for the rhesus leucocyte antigens (RhLA) -A, -B and -DR locus (Vreeseijk W. Van, et al., Tissue Antigens, 1977, 9: 17; Roger J.H., et al., J. Immunogen 1980, 7: 333)

### 2) mAbs used for in vivo treatment

Monoclonal antibodies (mAbs) used for in vivo treatment were designated MD1, 61E71 and H105-1.6. MD1 (murine IgG1) was raised against recombinant human IFN-γ (Meide et al Supra) and proved to fully neutralize the biological activity of naturally derived rhesus monkey IFN-γ. 61E71 (murine IgG₁) was raised against recombinant human TNF-α (Collins et al, Supra) and also proved to effectively neutralize rhesus monkey TNF-α. H105-1.6 is a murine anti-human mAb (IgG₂ₐ) with irrelevant specificity and not reacting with rhesus monkey antigens. All antibodies were purified from ascites by ammonium sulphate precipitation (50%) saturation). MD1 and 61E71 were also purified by adsorption to protein A. Prior to in vivo administration all mAbs were ultra centrifuged for 20 minutes at 20.000 x g and sterilized by millipore

filtration (0,22μM).

### 3) Skin grafting

Skin grafts were exchanged between non-immunized unrelated monkeys. Donor and recipients differed for all MHC antigens, both class I (RhLA-A and B) and Class II (RhLA-DR). Two grafts of about 2cm diameter were taken from the donors abdomen and, after removing the subcutaneous tissue, transplanted onto the back of the recipient. The grafts were fixed with sutures and a special dressing was used to immobilize the area and exert pressure on the grafts. From day 5 onwards, the skin grafts were inspected on alternate days by briefly opening and closing the dressing. On the 9th day after transplantation the dressing was permanently removed. After that time, grafts were examined twice a day and their condition was recorded until total rejection was observed. Major hemorrhages and crust formation over the entire graft was taken as the endpoint of graft survival (Balner, H, Transplantation 1969, 8: 206).

### 4) Experimental groups

The skin graft experiment concerned five different experimental groups. In group I, 10 animals did not receive any treatment. Five of these monkeys were historic controls (Vreeswijk, W., et al., Transplantation 1980, 30: 196).

In group II animals received H105-1.6. In this group skin graft recipients differed for one RhLA-A or -B antigen only. Results from group II have been published previously (Nooij, F.J.M., et al., Eur J. Immunol. 1987, 17: 1089). In group III and IV animals received either MD1 or 61E71. Animals in group V received the combination of MD1 and 61E71. Based on previous experiments, doses were used that would establish a small excess in serum levels of each injected antibody. Recipients received a daily dose of 1.3 mg/kg of MD1, 2.0 mg/kg of 61E71 or 1.0 mg/kg of H105-106 as an i.v. bolus injection for the duration of 10 days starting day -1, one day before skin transplantation.

### 5) Determination of level of injected mAb

In all groups serum levels of injected mAb were determined by ELISA. Flexible 96-well assay plates (Falcon 3911, Oxnard, CA) were coated overnight at 4°C with 25μl affinity purified goat anti-mouse IgG (Pel-Freeze Biologicals, Rogers. AR) at a concentration of 5μg/ml. Unoccupied binding sites were blocked by incubating the plates for 30 min at room temperature with 200μl phosphate buffered saline (PBS, RIA-grade, Sigma) containing 5% fetal calf serum (FCS). After washing, 20μl of 10-fold diluted serum samples were added per well and the plates were incubated for 2h at 37°C. After washing, the plates were subsequently incubated for 2h at 37°C, with 20μl affinity purified and peroxidase labelled goat anti-mouse IgG (Pel-Freeze). After another wash step, 50μl substrate solution was added, containing 2mg/ml O-phenylenediamine (Kodak, Rochester, NY) and 0.03% $H_2O_2$ in PBS. Color development was stopped by adding 25μl of 2N $H_2SO_4$. The plates were read at 492 nm. (Titertek Multiskan PLUS MKII, Mc Lean, Virginia). For quantitation of the amount of circulating mAb calibration curves were used of MD1 and 61E71 of a known concentration.

### 6) Determination of Monkey-anti-mouse IgG antibodies

The presence of anti-mouse IgG antibodies was determined in serum samples by a similar ELISA as described above. In this assay, plates were coated with MD1 or 61E71 (5μg/ml, 25μl/well). Tenfold dilutions of serum samples (20μl) were added per well. After extensive washing the plates were incubated with 20μl peroxidase-labelled rabbit anti-monkey IgG (H+L) serum (Nordic, Tilburg, The Netherlands). The plates were developed and read as described in section 5). The last ten-fold dilution that still showed a significant higher level of anti-mouse IgC antibodies to MD1 or 61E71 than the control, was taken as the titer of the sample.

### 7) Histological Studies

Biopsies were taken from the allogeneic skin grafts in group IV and V, at regular intervals after operation. After excision with a $3mm^2$ biospy stance a cylinder of full thickness skin was cut in half, longitudinally. One half was fixed in buffered formalin and processed routinely for histology on hematoxylin and azofloxine-stained sections. For parallel immunohistochemical studies the other half was snap-frozen in isopentane (-70°C). Cryostat sections of $6\mu l$ thickness were cut, air-dried, fixed in acetone and stored at -70°C until use. Incubation was performed using an indirect immunoperoxidase staining technique. Briefly, slides were thawed at room temperature for 15 min, washed with PBS, and then incubated with mAb for 60 min at room temperature. Slides were washed again and subsequently incubated for 30 min with horseradish peroxidase-coupled rabbit anti-mouse immunoglobulin (Dakopatts, Copenhagen, Danmark) diluted 1:100 in PBS containing 5% heat-inactivated (30 min., 56°C) normal rhesus serum and 5% normal rabbit serum. After rinsing, conjugate binding was visualised with a solution of 0.5 mg/ml 3,3,-diaminobenzidine tetrahydrochloride (Fluka Chemika, FRG) and 0.03% $H_2O_2$ in PBS. Slides were washed and counterstained with Mayer's hematoxylin for 1 minute, dehydrated and finally embedded in Malinol (Chroma gesellschaft, Kongen FRG).

The following antibodies were used for immunohistological staining: W6/32 (Serotec, Blackthorn, GB) specific for MHC class I antigens; anti-HLA-DR and anti-Leu6, respectively specific for MHC class II-DR antigens and CD1, present on Langerhans cells (both purchased from Becton and Dickinson, Mountainview, USA); the mAb T11 (DAKO-T11, Dakopatts, Danmark) specific for CD2, present on all rhesus T cells, as a pan T cell marker; a mixture of OKT4 and OKT4A (Ortho Pharmaceuticals Co., Raritan, NJ) specific for CD4+ cells and GM9 specific for CD8+ cells. GM9 was raised against rhesus monkey lymphocytes and produced at the TNO Primate center (Rijswijk, The Netherlands). All other mAbs are human specific but crossreactive in the rhesus monkey. In control incubations the primary antibody was omitted.

8) Assays for the determination of IFN-$\gamma$ and TNF-$\alpha$

In groups II to V, serum IFN-$\gamma$ levels were assessed semiquantitatively in a cytophatic inhibiton assay in HEp-2 cells with Vesicular Stomatitus Virus (VSV) as challenge (Meide et al., Supra). HEp-2 cells were seeded at a density of $2,5\times10^5$ cells per well in a 96-well microtiter plate in $100\mu l$ Dulbecco's modified Eagle's medium (Flow Laboratories, U.K.) containing 10% fetal calf serum (FCS). After 24h, at 37°C in a humidified $CO_2$ incubator, $100\mu l$ of two-fold serial dilutions was added and incubation was continued for 24h. Subsequently, $100\mu l$ of a VSV solution was added and incubation was continued for another 48h under similar conditions. Cell death was monitored with 0.5% crystal-violet staining.

Crossreactivity of the neutralizing activity of MD1 with IFN-$\gamma$ of the rhesus monkey has been determined in the same bioassay as described above, using naturally derived IFN-$\gamma$ from peripheral blood mononuclear cells of a rhesus monkey after stimulation with Concavaline A. One neutralizing unit (nu) is defined as that amount of pure anti-IFN-g mAb (MD1) that is able to neutralize one unit of antiviral activity induced by IFN-$\gamma$ as described previously (Meide et al., Supra)

The presence of biologically active TNF-$\alpha$ in serum was determined in a bioassay by measuring cell death of a murine WEH1 164 clone 13 fibrosarcoma cell line (Espevik T., et al, J. Immunol Methods (1986), 95:99). Briefly WEH1 164 cells were seeded at a density of $4 \times 10^4$ cells per well in 96-well microtiter plate in $100\mu l$ RPMI, containing 10% FCS. An equal volume of each serum sample was added in serial two-fold dilutions and incubated at 37°C for 24 hours in a humidified $CO_2$ incubator. Subsequently cell death was assessed with the tetrazolium salt method (Hansen, M.B., et al J. Immunol methods 1989, 199: 203).

Crossreactivity of the neutralizing activity of 61E71 towards TNF of different species origin was determined in the same assay, using naturally derived TNF from peripheral blood mononuclear cells of a rhesus monkey after stimulation with endotoxin (LPS Ecoli 055:B5, Sigma). The neutralizing capacity of 61E71 was defined as that amount of pure IgG that reduces the bioactivity of 3ng natural derived rhesus TNF one hundred fold.

Results

1) Effects of mAbs treatment on skin allograft survival

Untreated control animals had a mean skin graft survival time (MST) of 8.3 +/- 0.7 days (Table 1). MST were not prolonged by the administration of the irrelevant mAb H105-1.6 nor by MD1. The MST values in these groups were 8.2 and 9.5 days respectively. In the group consisting of animals treated with 61E71 alone a modest, non-significant prolongation of skin graft survival was observed (MST = 10 days) as compared with the untreated control group. However, prophylactic treatment with the combination of MD1 and 61E71 (group V) resulted in a significant prolongation of graft survival times to a MST of 12.3 days (Mann-Whitney U test p<0.05).

Table 1

| Skin allograft survival times in rhesus monkeys treated with mAbs specific for IFN-g and TNF-α | | | |
|---|---|---|---|
| Experimental groups (days) | Injected mAbs[a]) | Target | Individual skin graft survivial times |
| I | none | - | 7, 7.5, 8, 8, 8, 8, 9, 9, 9, 9 |
| II | H105-1.6[b]) | - | 8, 8, 8.5 |
| III | MD1 | IFN-$\gamma$ | 9, 9 |
| IV | 61E71 | TNF-$\alpha$ | 10, 10 |
| V | MD1 & 61E71 | IFN-$\gamma$ & TNF-$\alpha$ | 10.5, 12, 13, 13.5 |

a) Monoclonal antibody (mAb) treatment was started 1 day before skin grafting and continued for 10 days.
b) H105-1.6 is an irrelevant murine IgG2a monoclonal antibody.

2) Serum levels of antibody

In all cases, the daily injected doses of mAbs were sufficient to maintain detectable serum trough levels until about 1 day after the last injection. Levels, assessed by ELISA, varied from 35 ng/ml to 380μg/ml during 9 days after transplantation. On day 12 postoperatively, levels were all below detection level. No significant differences in mAb levels were observed between the different groups.

From these results it is clear that the augmented immunosuppresive effect of the combination of MD1 and 61E71 cannot be explained on basis of longer bioavailability of these mAbs in circulation compared to the groups where the mAbs were given separately. Furthermore, in all treated skin graft recipients, similar titers of anti-mouse-antibodies could be detected from 9 days after operation onwards. Thus, the aforementioned difference in MST between groups III, IV and V was not due to difference in immune reactivity towards the injected murine antibodies.

3) General histology

Histology was performed on skin grafts of monkeys in groups IV and V. At 5 days after transplantation, in all allogeneic skin grafts of group IV perivascular lymphocyte infiltrates became visible. With one exception, the skin grafts in the group of monkeys treated with both mAbs (group V), showed no or only very slight signs of rejection at day 5 postoperatively.

A 9 days after transplantation, in group IV the infiltrates of lymphocytes and macrophage-like cells became more diffuse throughout the graft. Capillary destruction and endothelial swelling of venes was present. In group V, however, lymphohistiocytic infiltrates remained of focal nature. Capillary destruction was less prominent and venes were not inflicted yet. The graft of one monkey in group V showed signs of venous changes already at 7 days after transplantation and 9 days postoperatively graft rejection was moderate as in the grafts of group IV.

After complete rejection, all skin grafts revealed necrosis of the superficial layers of the skin and crust formation.

In summary, in general the grafts of group V were at least two days later infiltrated by lymphocytes and macrophage like cells than the grafts in Group IV.

4) Immunohistology

At 5 days after operation, in all allogeneic skin biospies of group IV and V very small numbers of immunostained lymphocytes were found. The epidermis and the stroma of the dermis were faintly reactive with mAbs specific for MHC class I and II antigens and small numbers of Langerhans cells were stained with the mAb anti-Leu6.

At 9 days after operation, in group IV all preexisting structures in the papillary and reticulary dermis including vascular endothelium and the infiltrates showed very intense expression of MHC class I and II-DR antigens. Langerhans cells were stained strongly. Over 80% of the infiltrate was made up of T11+ cells. Less than 5% of these T cells were positive for OKT4 and 4A, implying that only very few CD4+ cells were present at the time of fullblown rejection. Almost all cells were positively stained with GM9, which defines the CD8+ phenotype. Similar changes were seen in the graft of one Group 5 monkey 9 days after operation. In all other grafts of the animals in group V, MHC class I and II antigen expression of preexisting structures was less prominent at this time. Infiltrates still had a perivascular localization and only 50 to 60% of each infiltrate was positive for T11. The ratio of CD4+ versus CD8+ cells was equally strongly in favour of CD8+ cells as in group IV.

After complete rejection, the epidermis and papillary dermis were necrotic and the intensity of staining for MHC class I and II antigens of the remaining structures of the dermis had diminished.

The immunohistology experiments thus confirm that the combination of antibodies delays the upregulation of MHC class I and II antigens.


5) Serum levels of IFN-γ and TNF-α

Only in an incidental case could the presence of IFN-g and TNF-α be established in serum, preoperatively. Furthermore, fluctuations in serum levels did not correlate with clinical events such as skin transplantation, administration of mAbs or rejection of the allograft (data not shown). These results suggest that the serum levels of these cytokines do not necessarily reflect the presence of these lymphokines on the site of allograft rejection, and that adminstration direct to a graft site, for example by topical administration would be suitable.


**Claims**

1. A pharmaceutical product containing an antibody to tumour necrosis factor (anti-TNF antibody) and an antibody to interferon-γ (anti-IFN-γ antibody) as a combined preparation for simultaneous separate or sequential use in therapy.

2. A pharmaceutical product according to Claim 1 wherein the preparation is for use in the therapy of an immunoregulatory disorder.

3. A pharmaceutical product according to Claim 1 or Claim 2 wherein the preparation is for use in the therapy of an auto-immune disease or organ or tissue transplant rejection.

4. A pharmaceutical product according to any one of the preceding claims comprising, in admixture, an anti-TNF antibody, an anti-IFN-γ antibody and, optionally, a pharmaceutically acceptable excipient, diluent or carrier.

5. Use of an antibody to TNF and an antibody to interferon-γ in the manufacture of a pharmaceutical product for use in therapy of an immunoregulatory disorder.

6. A use according to Claim 5 wherein the immunoregulatory disorder is an auto-immune disease, or organ or tissue transplant rejection.

7. A method for the production of a pharmaceutical product for use in therapy said method comprising arranging an anti-TNF antibody and an anti-IFN-γ antibody as a combined preparation for simultaneous separate or sequential use.

8. A method for the production of a pharmaceutical product according to any one of Claims 1 to 4, the method comprising mixing an anti-TNF antibody and an anti-IFN-γ antibody, and, optionally, a pharmaceutically acceptable excipient, diluent or carrier.

9. A method of treatment of an immunregulatory disorder in a human or animal subject, the method comprising administeringto said subject an anti-TNF antibody and an anti-IFN-γ antibody.

10. A product, method, or use according to any one of the preceding claims wherein the anti-TNF antibody is an antibody to human TNF-α

# PARTIAL EUROPEAN SEARCH REPORT

which under Rule 45 of the European Patent Convention
shall be considered, for the purposes of subsequent
proceedings, as the European search report

Application number

EP 90 30 .2556

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | JOURNAL OF IMMUNOLOGICAL METHODS, vol. 79, 1985, pages 293-305, Elsevier Science Publishers B.V. (Biomedical Division); P.H. VAN DER MEIDE et al.: "Monoclonal antibodies to human immune interferon and their use in a sensitive solid-phase ELISA" <br><br> * The whole article * <br><br>-- | 1-8,10 | C 12 P 21/08 <br> A 61 K 39/395 |
| D,Y | CANCER RESEARCH, vol. 48, October 1, 1988, pages 5411-5416; G. SANDRU et al.: "Stimulation of tumor cell growth in humans by a mononuclear cell-derived factor" <br><br> * The whole article * <br><br>-- | 1-8,10 | |
| | ./. | | **TECHNICAL FIELDS SEARCHED (Int. Cl.4)** <br><br> C 12 P <br> A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-8,10

Claims searched incompletely:

Claims not searched: 9

Reason for the limitation of the search:

Method for treatment of the human or animal body by surgery or therapy (see Art. 52(4) of the European Patent Convention).

| Place of search | Date of completion of the search | Examiner |
|---|---|---|
| THE HAGUE | 18-05-1990 | DIEDENHOFEN |

| DOCUMENTS CONSIDERED TO BE RELEVANT | | | CLASSIFICATION OF THE APPLICATION (Int Cl 4) |
|---|---|---|---|
| Category | Citation of document with indication, where appropriate, of relevant passages | Relevant to claim | |
| Y | HYBRIDOMA, vol. 6, no. 5, 1987, pages 489-507, Mary Ann Liebert, Inc., Publishers; T.S. BRINGMAN et al.: "Monoclonal antibodies to human tumor necrosis factors alpha and beta: application for affinity purification, immunoassays, and as structural probes" | | |
| | * The whole article * | 1-8,10 | |
| | -- | | |
| Y | WO-A-88 07 869 (STICHTING REGA VZW BE) | | |
| | * The whole document * | 1-8,10 | TECHNICAL FIELDS SEARCHED (Int Cl 4) |
| | -- | | |
| Y | WO-A-89 08 460 (CELLTECH LIMITED GB) | | |
| | * The whole document * | 1-8,10 | |
| | ---- | | |